# EUROPEAN PATENT APPLICATION

(11) **EP 0 960 621 A2**
(43) Date of publication of application: **01.12.1999**
(21) Application number: 99303559.1
(22) Date of filing: 06.05.1999
(51) Int. Cl.: A61K 31/505, A61K 9/20

(54) **Pharmaceutical formulations comprising sildenafil**

(30) Priority: 15.05.1998 US 85646 P
(71) Applicant: PFIZER INC., New York, N.Y. 10017 (US)
(72) Inventor: Bell-Huff, Cristi, West Bloomfield, Michigan 48322 (US); Dolan, Thomas Francis, Sandwich, Kent CT13 9NJ (GB); Hausberger, Angela Carol Gatlin, East Lyme, Connecticut 06333-1238 (US)
(74) Representative: Moore, James William

(57) **Abstract**

Pharmaceutical formulations of the compound sildenafil.

## Description

This invention relates to novel pharmaceutical formulations of the compound sildenafil and in particular to rapidly disintegrating oral dosage forms which contain sildenafil in the form of its free base.

The compound sildenafil (5-[2-ethoxy-5-(4-methylpiperazin-1-ylsulphonyl)-phenyl] - 1, 6- dihydro-1-methyl- 3- propylpyrazolo [4,3-d]pyrimidin-7-one) and its pharmaceutically acceptable salts are described and claimed in European patent EP-B-0463756. Their use in the treatment of male erectile dysfunction (impotence) and in female sexual disorders is disclosed and claimed in International patent application WO94/28902. Like most compounds being developed for human medicinal use, oral administration is preferred and for this purpose a soluble salt form was initially preferred to ensure rapid absorption and good bioavailability; this led to the selection of the citrate salt as being a highly soluble and non-toxic salt and this has been progressed into clinical trials and approved for marketing in certain countries.

While this has proved to be perfectly satisfactory for administration as a conventional capsule or tablet formulation, it was noted that the product did have an unpalatable bitter flavor.

For certain applications it is desirable to have a formulation that rapidly disintegrates in the mouth. An increase in the disintegration rate in the mouth facilitates administration to certain patients and can improve the rate of absorption of the active ingredient. Examples of tablets which are porous and fast-disintegrating are described in US patent numbers 3,885,026, and 4,134,943. Porous and fast dispersing tablets having increased strength are described in European patent EP-B-0620728. Such tablets are formed by using a meltable binder, a disintegrating agent and a volatilizable component which is removed from the tablet after compression of the tablet ingredients to form a porous tablet.

One problem with the rapidly dispersing solid dosage forms described above is that the patient will taste the pharmaceutically active substance as the dosage form disintegrates in the mouth. For some pharmaceutically active substances, the taste, if only slightly unpleasant, can be rendered acceptable by the use of sweetening agents or flavoring agents to mask the taste. However, this technique does not completely mask the taste of sildenafil citrate. Another way of taste-masking is by microencapsulation using a polymer coating which protects the active ingredient while in the mouth; however this technique is complex and expensive and can give rise to bioequivalency problems. WO96/13252 describes an alternative approach by using a form of the pharmaceutically active substance which is less soluble in water. While this approach is possible in theory, in practice it is often difficult to find a form of the active ingredient which is sufficiently insoluble to be completely tasteless in the mouth, as the tongue can detect even small amounts of materials, particularly if these have a strong flavor. Moreover it is also necessary that the insoluble form of the product dissolves once it has been swallowed in order for it to be absorbed into the bloodstream.

According to the present invention, we have surprisingly discovered that sildenafil, in the form of its free base has extremely low solubility in water, and in saliva, and this makes it particularly suitable for use in orally dispersible formulations, being virtually tasteless. Moreover we have now unexpectedly discovered that despite the low solubility of sildenafil free base, it is efficiently absorbed from the stomach or gastrointestinal tract and such formulations can provide blood plasma levels of the active ingredient which are virtually identical to those achieved with oral solutions of sildenafil citrate or with the conventional tablet or capsule formulations of sildenafil citrate.

Thus according to the present invention there is provided an orally disintegrating pharmaceutical preparation which comprises sildenafil free base together with a pharmaceutically acceptable carrier.

Particularly preferred are orally disintegrating pharmaceutical preparations which comprise sildenafil free base together with a pharmaceutical carrier which rapidly disintegrates in the mouth.

By the term "rapidly disintegrating" in respect of oral pharmaceutical preparations as used herein we mean a tablet, wafer or other solid dosage form which will disintegrate in water at 37°C within a period of 60 seconds or less, preferably 5 to 10 seconds or less. The disintegrating ability of any particular formulation may be tested by standard pharmaceutical methods, using for example the procedure described in WO96/13252 which is analogous to the Disintegration Test for Tablets, B.P. 1973 which is described in British Patent No. 1548022. The procedure is included hereafter for completeness.

In a particular embodiment of the invention a fast dispersing oral solid dosage form containing sildenafil free base may be prepared using a water-soluble binder and a volatilizable component which is removed from the tablets after combination and compression of the tablet ingredients to provide highly porous tablets.

In this embodiment, the tablet is prepared according to the procedure of EP-B-0620728. This procedure requires the steps of (a) combining and compressing a water soluble meltable binder, at least one excipient, and sildenafil free base into a tablet, (b) melting said binder in said tablet, and (c) solidifying said binder.

The water soluble meltable binder which is used to increase the strength of the final tablet has a melting point generally ranging from 20°C to 100°C, preferably from 40°C to 70°C. The melting point of the binder is usually above 20°C since the mixing of the ingredients is usually carried out at this temperature and the binder should be solid at the mixing temperature. Of course, if mixing is carried out at lower temperatures, a lower melting binder may be used which is solid at that temperature.

The melting point of the binder is usually not higher than 100°C since the melting of the binder should be at a temperature at which the activity of the pharmaceutically active agent is not adversely affected. For instance, the melting of the binder should be at a temperature lower than the decomposition temperature of the pharmaceutically active agent as well as any of the excipients present.

The amount of the water soluble meltable binder in the tablet of this embodiment of the invention typically ranges from 5% to 40% by weight, preferably from 8% to 25% by weight, based on the weight of the tablet. Excessive amounts of the water soluble meltable binder should be avoided as the tablet may deform during melting whereas if insufficient amounts are used the desired strength may not be attained.

Suitable water soluble meltable binders include polyethylene glycols (PEG) having molecular weights ranging from about 1,500 to about 20,000 such as PEG 3350 (m.p. 58°C) and PEG 8000 (m.p. 62°C), sucrose esters such as sucrose monostearate (m.p. 49-56°C), and sucrose monopalmitate (m.p. 40-48°C), ethoxylated fatty acids such as polyoxyethylene (40) stearate (m.p. 47°C) (Myrj-52S), and ethoxylated alcohols such as polyoxyethylene (23) lauryl ester. These meltable water-soluble binders enable the preparation of tablets having increased disintegration rates in the mouth, e.g., rapid disintegration rates of less than ten seconds, and as low as from 1 to 5 seconds.

The water soluble meltable binder may be combined with the excipient or excipients and the pharmaceutically active agent in any sequence. The binder may be combined in dry form or in a suitable solvent such as alcohol, isopropanol or water. The dissolved binder on addition to the remaining tablet ingredients forms a wet granulation. If desired, the sildenafil is added after drying of the wet granulation. Usually, the combined tablet ingredients are milled and mixed with a tabletting lubricant before the dry granules are compressed into tablets.

The excipients used in the tablet are generally known in the art, i.e., as described in Remington's Pharmaceutical Sciences, 18th Edition (1990), particularly pages 1633 to 1638. They impart necessary processing and compression characteristics either to the tablet formulation before tabletting, or to the finished tablet. Examples of excipients are diluents, binders, lubricants, flavors, and sweetening agents. Specific diluents of use in the invention are water soluble diluents such as mannitol, xylitol, sucrose, lactose, and sodium chloride. Suitable binders of use in the invention, in addition to the water soluble meltable binder of use in the invention, impart cohesive properties and include starch, gelatin, microcrystalline cellulose and sugars such as sucrose, glucose, dextrose, and lactose. As is clear from the above, the same excipient may be used for different purposes within the same tablet formulation.

A disintegrating agent may be present to increase the disintegration rate of the tablet after oral intake. Examples of disintegrating agents are cellulose such as carboxymethylcellulose, starches, clay, algins, gums and crosslinked polymers, such as crosslinked polyvinylpyrrolidone (PVP-XL).

In the embodiment of this aspect of the invention, a volatilizable component is present in the tablet formulation. After combination and compression of the tablet ingredients, the volatilizable component is removed from the tablets by heating at atmospheric or reduced pressure to form porous tablets. In a preferred process, tablets are heated to 50-60°C under a continuous nitrogen purge until the volatile component is completely removed via sublimation. Using a nitrogen purge helps protect against degradation of sildenafil under these conditions, however, an air purge may be used for temperatures of 50-55°C. Suitable volatilizable components include sublimable materials such as menthol, camphor, urea, and vanillin, and materials that decompose at or below the melting point of the tablet binder such as ammonium bicarbonate. The amount of volatilizable material ranges from 1% to 95% by weight, based on the weight of the combined tablet ingredients. For instance, when using ammonium bicarbonate, the amount is usually from 50% to 90% by weight, and when using menthol, the amount typically ranges from 30% to 55% by weight. Preferably, the volatilizable material is removed during the melting step when the compressed tablets are heated above the melting point of the meltable binder for a period of time sufficient to melt the meltable binder and to remove the volatilizable material. When using menthol, removal thereof is by heating to about 40°C under vacuum; with ammonium bicarbonate removal is effected by heating under vacuum at 60°C.

Tablets prepared by any of these methods may be coated with a thin layer of a coating material to improve the surface integrity of the tablet. Suitable coating materials include disaccharides such as sucrose, polysaccharides such as maltodextrins and pectin, and cellulose derivatives such as hydroxypropylmethylcellulose and hydroxypropylcellulose, however any such coating should be sufficiently thin and water soluble as to not interfere with the ability of the tablet to disintegrate rapidly in the mouth.

The invention will now be more particularly described with reference to the following examples:

### EXAMPLE 1

A highly porous tablet which disintegrates rapidly in the mouth was prepared following the procedures of EP-B-0620728 as follows:

Ingredients 2, 3, 4, and 5 were blended and wet-granulated using ethanol. The dry granules were milled and blended with ingredients 1, 6, 7, 8, 9, and 10. Finally, ingredient 11 was mixed with the blend. The final blend was compressed into tablets of 12.7 mm (0.5 inch) diameter. These tablets were then heated at 50-60°C under a continuous nitrogen purge until the ammonium bicarbonate was completely sublimed.

| Ingredients | | mg/tablet |
|---|---|---|
| 1 | Sildenafil (free base) | 50.00 |
| 2 | Ammonium bicarbonate | 308.00 |
| 3 | Mannitol | 44.7 |
| 4 | Polyethylene Glycol 3350 | 5.6 |
| 5 | Hydroxypropyl cellulose | 11.2 |
| 6 | Sodium saccharin | 3.6 |
| 7 | Compressible sugar | 19.00 |
| 8 | Silicon dioxide | 2.4 |
| 9 | Banana flavor | 23.90 |
| 10 | Sodium stearyl fumarate | 4.8 |
| 11 | Magnesium stearate | 4.8 |

Notes:
(1) Pore former, not present in final dosage form.
(4) PEG 3350 a soluble polyethylene glycol having m.p. 58°C used as the meltable binder in the formulation.
(11) Tablet lubricant.

The tablet had a highly porous structure which disintegrated rapidly in the mouth on oral administration and had an acceptable taste.

### EXAMPLE 2

The procedure of Example 1 is followed using quantities of excipients selected from the following ranges:

| Ingredients | | mg/tablet |
|---|---|---|
| 1 | Sildenafil (free base) | 50 |
| 2 | Ammonium bicarbonate | 240-360 |
| 3 | Mannitol | 40-100 |
| 4 | Polyethylene Glycol 3350 | 5-20 |
| 5 | Hydroxypropyl cellulose | 0-15 |
| 6 | Sodium saccharin | 0-6 |
| 7 | Compressible sugar | 0-40 |
| 8 | Silicon dioxide | 0-5 |
| 9 | Banana flavor | 0-50 |
| 10 | Sodium stearyl fumarate | 2-7 |
| 11 | Magnesium stearate | 2-7 |

Tablets having a highly porous structure which disintegrate rapidly in the mouth on oral administration and have an acceptable taste are obtained.

### EXAMPLE 3

The procedure of Example 1 is followed using quantities of excipients selected from the following ranges:

| Ingredients | | mg/tablet |
|---|---|---|
| 1 | Sildenafil (free base) | 50 |
| 2 | Ammonium bicarbonate | 240-360 |
| 3 | Mannitol | 40-100 |
| 4 | Polyethylene Glycol 3350 | 5-20 |
| 5 | Hydroxypropyl cellulose | 0-15 |
| 6 | Sodium stearyl fumarate | 2-7 |
| 7 | Magnesium stearate | 2-7 |

Tablets having a highly porous structure which disintegrate rapidly in the month on oral administration and which have an acceptable taste are obtained.

### Measurement of Disintegration Rates

### Apparatus

A glass or suitable plastic tube 80 to 100 mm long, with an internal diameter of about 28 mm and an external diameter of 30 to 31 mm, and fitted at the lower end, so as to form a basket, with a disc of rustproof wire gauze complying with the requirements for a No. 1.70 sieve (B.P. 1973 page A136).

A glass cylinder with a flat base and an internal diameter of about 45 mm containing water and not less than 15 cm deep at a temperature between 36° and 38°C.

The basket is suspended centrally in the cylinder in such a way that it can be raised and lowered repeatedly in a uniform manner so that at the highest position the gauze just breaks the surface of the water and at the lowest position the upper rim of the basket just remains clear of the water.

### Method

Place one dosage form in the basket and raise and lower it in such a manner that the complete up and down movement is repeated at a rate equivalent to thirty times a minute. The dosage form is disintegrated when no particles remain above the gauze.

## Claims

1. An orally disintegrating pharmaceutical preparation which comprises sildenafil free base together with a pharmaceutically acceptable carrier.

2. An orally disintegrating pharmaceutical preparation as claimed in claim 1 which comprises sildenafil free base together with a pharmaceutical carrier, which rapidly disintegrates in the mouth.

3. A preparation as claimed in claim 2, wherein said preparation is prepared by a method which comprises the steps of
(a) combining and compressing a water soluble meltable binder, at least one excipient and sildenafil free base into a tablet,
(b) melting said binder in said tablet and
(c) solidifying said binder.

4. A preparation as claimed in claim 3 wherein said binder is a polyethylene glycol, a sucrose ester, an ethoxylated fatty acid or an ethoxylated alcohol.

5. A preparation as claimed in claim 3 wherein step (a) includes combining with a volatilizable material which is removed after the compression step.

6. A preparation as claimed in claim 5 wherein the volatilizable material is ammonium bicarbonate.

7. A method of treating human sexual dysfunction which comprises administering an orally disintegrating pharmaceutical preparation as claimed in claim 1, and allowing said preparation to disintegrate in the mouth.

8. A method as claimed in claim 7 wherein the pharmaceutical preparation is administered to an impotent male.

9. A method as claimed in claim 7 where the pharmaceutical preparation is administered to a female.
